# EUROPÄISCHE PATENTSCHRIFT

(11) **EP 2 605 731 B1**
(45) Veröffentlichungstag und Bekanntmachung des Hinweises auf die Patenterteilung: **01.04.2015**
(21) Anmeldenummer: 11746480.0
(22) Anmeldetag: 17.08.2011
(51) Int. Cl.: A61F 5/02

(54) **SPANNVORRICHTUNG FÜR ORTHESEN**
FASTENING DEVICE FOR ORTHOSES
DISPOSITIF TENDEUR POUR ORTHÈSES

(30) Priorität: 18.08.2010 DE 102010035309
(43) Veröffentlichungstag der Anmeldung: 26.06.2013
(73) Patentinhaber: Bauerfeind AG, 07937 Zeulenroda-Triebes (DE)
(72) Erfinder: STIER, Gerald, 07937 Langenwetzendorf (DE)
(74) Vertreter: Wohlfahrt, Jan Günther
(86) Internationale Anmeldenummer: PCT/EP2011/004125
(87) Internationale Veröffentlichungsnummer: WO 2012/022470

(56) Entgegenhaltungen:
- GB-A- 2 086 712
- US-A1- 2006 174 459
- US-A1- 2010 168 630
- US-B1- 6 213 968

## Beschreibung

Die Erfindung betrifft eine neuartige Spannvorrichtung für Orthesen zur Stützung und Funktionserhaltung des menschlichen Körpers, besonders körperumgreifende Rückenorthesen. Die Spannvorrichtung erlaubt ein individuelles, segmentweises Anpassen der Stützwirkung durch individuell separat ausgebildete Flaschenzüge.

Orthesen sind therapeutische Hilfsmittel zur Stabilisierung oder Unterstützung der Bewegungsfunktion von Körperteilen, beispielsweise von Becken und Wirbelsäule. Der Einsatz der Orthesen kann direkt posttraumatisch oder postoperativ oder konservativ erfolgen. Bei der Verwendung werden die Orthesen in der Regel um das Körperteil, beispielsweise um die Hüfte herum, angelegt und so gürtelartig geschlossen, dass ein stabilisierender Druck auf die zu stabilisierende Körperregion ausgeübt wird. Beispielsweise bei Lumbalorthesen kann es erforderlich sein, eine bestimmte Wirbelsäulenkrümmung (Lordose) zu fixieren, um weitere Schäden an der Wirbelsäule zu verhindern oder einen postoperativen Zustand zu stabilisieren, um die Heilung zu verbessern.

Bekannte Orthesen, beispielsweise Lumbalorthesengürtel, weisen häufig Spannvorrichtungen auf, mit deren Hilfe die Spannung/der Druck der umgelegten Orthese auf das Körperteil kontrolliert erhöht werden kann. Dazu ist häufig eine Flaschenzugvorrichtung vorgesehen. Diese erstreckt sich bekanntermaßen im Wesentlichen über die gesamte Breite der Orthese mit dem Ziel, die Zugkraft über die gesamte Breite des Orthesengürtels zu vergleichmäßigen. Bekannte, auf Flaschenzügen basierende Spannvorrichtungen weisen dazu ein loses Zugseil auf, das zu einer Seite der Orthese hin verläuft. Es hat sich herausgestellt, dass derart aufgebaute Orthesen bei der Verwendung mit Nachteilen behaftet sind: Durch die durch den bekannten Flaschenzugmechanismus bedingte Vergleichmäßigung der Zugkraft ist eine individuelle Anpassung der Spannung und damit der Stützwirkung auf ein individuelles Behandlungsziel nicht möglich. Gleichzeitig kommt es durch das Spannen bekannter Flaschenzugvorrichtungen über einseitige Zugseile zu einer einseitigen Kraftwirkung an der Orthese und damit zu deren Verziehen oder Verrutschen.

Die Erfindung hat sich die Aufgabe gestellt, Spannvorrichtungen für Orthesen weiterzuentwickeln und zu verbessern, um die bekannten Nachteile zu vermeiden. Das der Erfindung zugrunde liegende technische Problem liegt in der Bereitstellung einer verbesserten Spannvorrichtung für eine Orthese, die insbesondere in ihrer Stützfunktion besser an die Anforderungen der Therapie oder Prophylaxe angepasst werden kann. Gleichzeitig soll die Vorrichtung leichter und zuverlässiger bedienbar und einstellbar sein; ein Verrutschen oder Verwinden soll vermieden werden.

Das technische Problem wird vollständig gelöst durch eine Spannvorrichtung für eine Orthese, die vor allem dadurch gekennzeichnet ist, dass sie mehrere getrennte, separate, besonders im Wesentlichen parallel verlaufende, zueinander benachbarte, besonders unmittelbar angrenzende im Wesentlichen gleichartige Spannsegmente aufweist. Diese Spannsegmente sind voneinander unabhängig über separate Flaschenzüge spannbar.

Die Spannsegmente weisen jeweils ein erstes Seitenelement an einem ersten Ende der Spannvorrichtung und ein zweites Seitenelement an einem gegenüberliegenden zweiten Ende der Spannvorrichtung auf. Dazwischen ist ein bevorzugt mittig zwischen dem ersten und zweiten Seitenelement ein Zentralelement angeordnet. Die Seitenelemente sind über das Zentralelement miteinander mechanisch verbunden, und zwar über jeweils einen zwischen dem Zentralelement und dem ersten Seitenelement verlaufenden ersten Flaschenzug und einen zwischen dem Zentralelement und dem zweiten Seitenelement verlaufenden zweiten Flaschenzug, und bilden so ein Spannsegment.

Im angelegten Zustand ist die Spannvorrichtung zusammen mit der Orthese um das Körperteil herum gelegt und die beiden Enden der Spannvorrichtung stehen miteinander in kraftschlüssiger Verbindung; die Spannvorrichtung ist wie ein Gürtel um das Körperteil geschlossen. Die Erfindung sieht innerhalb eines einzelnen Spannsegments zu beiden Seiten eines Zentralelements jeweils einen Flaschenzug vor, der sich zu den Seitenelementen hin erstreckt. Die sich gegenüber liegenden Flaschenzüge eines Spannsegments können bei der Benutzung der angelegten Orthese über ein beidseitiges Ziehen der losen Enden ihrer beiden Zugseile in entgegengesetzter Richtung zirkulär gespannt werden. Beim Spannen wird der Abstand zwischen den Seitenelementen und damit den beiden Enden der Spannvorrichtung zueinander zu verkürzt und eine das Körperteil umlaufende zirkuläre Spannung erzeugt. Dadurch wird vorteilhafterweise eine zum Zentralelement symmetrische Krafteinwirkung erreicht. Die zirkuläre Spannung kann so unmittelbar symmetrisch gleichmäßig auf beiden Seiten des Zentralelements wirken, sodass dieses beim Spannen nicht aus seiner Position verschoben wird. Ein Verwinden oder ein Verschieben der Spannvorrichtung oder der mit der Spannvorrichtung verbundenen Orthese beim Spannen wird wirksam verhindert.

In einer erfindungsgemäßen Ausgestaltung sind Flaschenzüge mit mehrfacher Umlenkung ausgebildet. Die Flaschenzüge sind jeweils an dem Zentralelement und an dem ersten beziehungsweise zweiten Seitenelement ein und desselben Spannsegmentes verankert. Der Flaschenzug eines Spannsegments hat keine Verankerung oder Umlenkpunkte an einem anderen insbesondere benachbarten Spannsegment. Erfindungsgemäß weist also jedes Spannsegment eigene, zu den anderen Spannsegmenten getrennte Flaschenzüge auf.

In Abkehr vom Stand der Technik sieht die Erfindung also auch vor, mehrere Flaschenzüge vorzugsweise im Wesentlichen parallel zueinander unabhängig in separaten Spannsegmenten anzuordnen. Jeder Flaschenzug greift jeweils ausschließlich an den ihm zugeordneten Zentralelement und Seitenelementen eines Spannsegments an. Jedem einzelnen Spannsegment ist also in dieser Ausgestaltung nur ein einziges individuell einstellbares Paar von Flaschenzügen zugeordnet. Jeder Flaschenzug ermöglicht in Verbindung mit dem ihm gegenüberliegenden, zum anderen Ende hin weisenden Flaschenzug eine segmentweise individuell einstellbare symmetrische zirkuläre Spannwirkung.

Die Erfindung sieht besonders vor, dass zur Realisierung des Zugseilantriebs in Form eines Flaschenzugs an dem Zentralelement zu beiden Seiten hin pro Segment jeweils, bevorzugt genau ein Zugseilumlenkelement zum Umlenken des Zugseils des jeweiligen Flaschenzugs oder wahlweise oder zusätzlich, bevorzugt genau eine Verankerung des Zugseilendes vorgesehen ist. In Abkehr vom Stand der Technik vermeidet die Erfindung die Vergleichmäßigung der Kraftwirkung eines Flaschenzugs, über größere Abschnitte beziehungsweise die gesamte Breite des Orthesengürtels bei bekannten, mehreren nebeneinander liegenden Zugseilumlenkelementen oder Verankerungen des Zugseils. Vielmehr besitzt der erfindungsgemäße Flaschenzug bevorzugt jeweils genau einen Verankerungspunkt mit dem Zentralelement und dem Seitenelement des Spannsegments; der Verankerungspunkt ist jeweils als Zugseilumlenkelement, bevorzugt als Umlenkrolle, oder als Verankerungspunkt, worin das Zugseil ortsfest fixiert ist, ausgebildet. Erfindungsgemäß wird also pro Spannsegment jeweils in einem einzigen Kraftansatzpunkt über den Flaschenzug Kraft ausgeübt. Der Kraftansatzpunkt ist, je nach Auslegung des Flaschenzugs, als Zugseilumlenkung oder, alternativ oder zusätzlich, als Zugseilverankerung ausgebildet. Es ist in einer besonderen Ausführung der Erfindung vorgesehen, dass an Zentralelement und/oder Seitenelement mindestens zwei beabstandete optionale Verankerungspunkte vorbereitet sind, die zur individuellen Anpassung der Spannvorrichtung jeweils als alternative Kraftansatzpunkte gewählt werden können.

In besonderer Ausgestaltung weist die Spannvorrichtung zumindest zwei, bevorzugt drei oder vier oder fünf, separat spannbare Spannsegmente auf. Bevorzugte Varianten weisen genau drei, vier oder fünf aneinander angrenzende Spannsegmente auf, denen jeweils ein Flaschenzugpaar zugeordnet ist. Diese Varianten sind also an drei, vier oder fünf Segmenten separat spannbar.

Die segmentweise Krafteinleitung in einem einzigen Kraftansatzpunkt innerhalb eines Segments erlaubt, vor allem im Zusammenhang mit der Verwendung der Spannvorrichtung an oder in einer Rückenorthese eine wirbelsegmentsgenaue Krafteinleitung. Vorzugsweise sind in dieser Ausgestaltung der Erfindung einem Spannsegment zumindest ein Wirbel oder eine Wirbelgruppe zugeordnet. Bevorzugt bewirkt ein einzelnes Spannsegment eine überwiegende Krafteinwirkung an oder im Bereich genau eines Wirbels oder Wirbelabschnitts und ein anderes Spannsegment bewirkt eine überwiegende Krafteinwirkung an oder im Bereich genau eines anderen Wirbels oder Wirbelabschnitts.

In einer besonderen Ausgestaltung ist zusätzlich vorgesehen, dass zur erfindungsgemäßen segmentweisen Krafteinwirkung mindestens ein Spannsegment zusätzlich, bevorzugt an oder im Bereich seines Zentralelements, mindestens eine zum Körperteil gewandte Pelotte aus bevorzugt elastischem Polstermaterial aufweist. Diese Pelotte ist bevorzugt derart ausgestaltet, dass sie die Krafteinwirkung dieses Spannsegments gezielt auf eine bestimmte Körperregion richtet. In einer bevorzugten Variante die Pelotte an der Spannvorrichtung individuell ab- oder aufrüstbar. Die Pelotte ist bevorzugt in ihrer Form und/oder ihrem Werkstoff zur Modifikation der Wirkungsweise der Vermittlung der an dem Spannsegment austausch und/oder einstellbar. In einer besonderen Variante kann einem Spannsegment eine Pelotte zugeordnet sein, die bestimmte Weichteilstrukturen des Körperteils, in Verbindung mit der gezielt einstellbaren Krafteinwirkung des Spannsegments, individuell massiert und stimuliert. Beispielsweise kann so ein Triggerpunkt eines Muskels gezielt stimuliert werden, um beispielsweise eine gezielte Spannung oder Entspannung des Muskels zu erreichen. Solche gezielt segmentweise einstellbaren und auslösbaren Einwirkungen auf das Körperteil sind durch bekannte spannbare Orthesen nicht erreichbar.

In einer bevorzugten Ausgestaltung weist das Zentralelement für die Flaschenzüge an einem Kraftansatzpunkt sowohl eine Zugseilverankerung für das Zugseil als auch eine Zugseilumlenkung als zweite Umlenkung auf. Entsprechend weisen dabei die Seitenelemente als Kraftansatzpunkt eine Zugseilumlenkung als Umlenkung des Zugseils auf, um einen dreifachen Flaschenzug zu bilden. Die Erfindung betrifft selbstverständlich auch andere konkrete Ausgestaltungen von Flaschenzügen mit einer Verankerung und einer oder mehrerer Umlenkungen. Sind in dem einen Kraftansatzpunkt des Spannsegments, sei es an Zentralelement oder dem Seitenelement, mehrere Zugseilumlenkungen vorgesehen, so ist bevorzugt, dass die Umlenkungen als gestapelte Umlenkrollen mit gemeinsamer Achse in dem einzigen Kraftansatzpunkt einen Rollenblock bilden.

In besonderer Ausgestaltung ist zusätzlich vorgesehen, dass zumindest zwei benachbarte Spannsegmente der Spannvorrichtung miteinander mechanisch verbunden sind. Hierbei ist bevorzugt eine starre Kopplung vorgesehen. Alternativ bevorzugt ist eine flexible Kupplung, besonders in Form eines flexiblen, beispielsweise elastischen, Bandes. In besonderer Ausgestaltung davon sind zumindest die Seitenelemente zweier unmittelbar benachbarter Segmente mechanisch miteinander verbunden, um mindestens eine integrale Seitenbrücke aus mindestens zwei Seitenelementen zu bilden. In einer besonderen Ausgestaltung sind die Seitenelemente aller vorhandenen Segmente miteinander in dieser Weise zu einer einzigen Seitenbrücke verbunden. Die Seitenbrücken sind in einer besonderen Variante einstückig ausgebildet.

In besonderer Ausgestaltung davon sind, alternativ oder zusätzlich, zumindest die Zentralelemente zweier unmittelbar benachbarter Segmente mechanisch miteinander verbunden, um mindestens eine integrale Zentralbrücke aus mindestens zwei Zentralelementen zu bilden. In einer besonderen Ausgestaltung sind die Zentralelemente aller vorhandenen Segmente miteinander in dieser Weise zu einer einzigen Zentralbrücke verbunden. Die Zentralbrücke kann einstückig ausgebildet sein.

In besonderer Ausgestaltung weist die integrale Zentralbrücke zusätzlich mindestens ein senkrecht zur Kraftrichtung der Flaschenzüge verlaufendes Stützelement für ein Körperteil auf oder es besteht daraus. Dieses Stützelement ist an oder in dem oder insbesondere als Zentralelement ausgebildet. Das Stützelement ist aus einem vergleichsweise unelastischen zähen und spröden Werkstoff ausgeführt. Das Stützelement kann in Form eines einfachen Stabes, beispielsweise als individuell formbarer Metallstab oder thermoplastisch formbarer Kunststoffstab ausgebildet sein. Durch die erfindungsgemäß segmentweise ausübbare Spannkraft kann dieses Stützelement individuell an das Körperteil angelegt werden, um die Stützfunktion beziehungsweise die therapeutische formgebende Funktion zu ermöglichen. Besonders ist dieses Stützelement in anatomisch und therapeutisch zweckmäßiger Weise geformt und dient unmittelbar der Stützung eines Körperteils, besonders der Wirbelsäule, das heißt im Beispiel einer Rückenorthese verläuft das Stützelement entlang der Wirbelsäule und dient damit zu deren Stützung.

Alternativ sind anstelle eines einzelnen Stützstabes zwei zueinander beabstandete stabförmige Stützelemente, die über Querbrücken, die zwischen Aussparungen des Zentralelements gebildet sind, miteinander verbunden sind, vorgesehen. Besonders im Falle einer Rückenorthese kann so die Wirbelsäule unmittelbar links und rechts der Wirbelkämme gestützt werden; eine unmittelbare Druckbelastung der Wirbelkämme wird vermieden.

Die Erfindung sieht vor, dass die Zugseile des Flaschenzugs eines ersten Spannsegments jeweils zusammen, aber getrennt und beabstandet von den Zugseilen des Flaschenzugs eines benachbarten zweiten Segments geführt werden. In einer besonderen Ausgestaltung ist dazu ein Gitterrahmen vorgesehen, der jeweils zwischen den Zentralelementen und Seitenelementen angeordnet ist, und die Zugseile eines Flaschenzugs zusammen und jeweils getrennt von den Zugseilen eines anderen Flaschenzugs beabstandet hält. Die Zugseile sind dazu entsprechend durch die Maschen des Gitterrahmens geführt. Dabei ist bevorzugt vorgesehen, dass, besonders im Falle einer Rückenorthese, der Gitterrahmen gleichzeitig als stabilisierende Beckenfassung ausgebildet ist, der gleichzeitig rotationsstabilisierend wirkt.

In besonderer Ausgestaltung der Erfindung werden die Zugseile eines Flaschenzugs in einem Zugseiltunnel geführt. Der Zugseiltunnel erstreckt sich bevorzugt zwischen dem Seitenelement und dem Zentralelement. Der Zugseiltunnel ist bevorzugt aus einem plastischen flexiblen Werkstoff ausgeführt; besonders bevorzugt ist ein elastisches Gestrick. Die Erfindung ermöglicht so eine kompakte und anwenderfreundliche Realisierung der segmentweisen individuellen Flaschenzüge. Ein Inkontaktbringen der Zugseile benachbarter Flaschenzüge und ein Verhaken mit Kleidungsstücken oder anderen Orthesenbestandteilen wird so wirkungsvoll verhindert.

In einer besonderen Variante dieser Ausgestaltung weist der Zugseiltunnel zusätzlich einen Tunnelzweig auf, der im Verlauf zwischen Seitenelement und Zentralelement abzweigt. Darin kann jeweils das lose Ende des Zugseils eines Flaschenzugs geführt werden. Durch den ebenso wie der Zugseiltunnel aus plastischem oder flexiblem Werkstoff ausbildbaren Tunnelzweig kann das lose Ende so geführt werden, dass die Gefahr des Verdrillens oder Verknotens mit den Zugseilenden benachbarter Flaschenzüge verhindert und die Anwendung der Spannvorrichtung beim Anlegen und Spannen vereinfacht wird. In einer alternativen oder zusätzlichen Ausgestaltung werden die Zugseile der Flaschenzüge jeweils getrennt in einem Abstandsgewirk geführt.

In einer besonderen Ausgestaltung sind die Zugseile unmittelbar in das Gestrick einer Orthese eingelegt. Dazu weist das Gestrick der Orthese vorzugsweise tunnelartige Aussparungen oder Laschen auf, die eine getrennte Führung der Zugseile ermöglichen. Zusätzliche Maßnahmen wie der in der vorbezeichneten Ausgestaltung vorgesehene Gitterrahmen zur Beabstandung der Zugseile benachbarter Flaschenzüge erübrigen sich in dieser Ausgestaltung. In der vereinfachten Ausgestaltung einer bereits in die Gestrickorthese integrierten erfindungsgemäßen Spannvorrichtung ist eine hierin beschriebene Auf- und Abrüstbarkeit der Spannvorrichtung auf eine stehende, insbesondere herkömmliche Orthese, besonders Gestrickorthese, nicht erforderlich; die erfindungsgemäße Spannvorrichtung ist vielmehr ein integraler Bestandteil einer entsprechenden neuartigen hierin beschriebenen erfindungsgemäßen Orthese.

In einer besonderen Ausgestaltung mündet das lose Ende eines Zugseils in einen Griff, worin es fixiert ist. Über den Griff kann der Benutzer das lose Ende des Zugseils eines Flaschenzugs spannen. Es ist vorgesehen, dass der Griff im Bereich des mit dem Flaschenzug verbundenen Seitenelements, das heißt im Bereich der jeweiligen Enden der Spannvorrichtung, lösbar fixierbar ist, um die Spannung aufrecht zu erhalten. Zum Voreinstellen der Zuglänge des Zugseilendes kann das Griffstück eine Klemmvorrichtung aufweisen. Hiermit kann der "Arbeitspunkt" jedes Flaschenzugs segmentweise eingestellt werden, um die individuelle Anpassung der Orthese sicherzustellen. Die zeitweise Fixierung des Griffs an der Spannvorrichtung erfolgt in an sich bekannter Weise über Verklettung oder Verknüpfung.

In einer besonderen Variante sind jeweils die losen Enden der Zugseile der Flaschenzüge benachbarter Spannsegmente in einen gemeinsamen Griff geführt und darin zueinander beabstandet fixiert. Die Erfindung sieht in dieser Ausgestaltung vor, dass durch Schwenken oder Neigen des Griffs der Zug auf das eine oder andere Zugseil individuell bestimmt werden kann. Dazu ist bevorzugt vorgesehen, dass der gemeinsame Griff in dem verschwenkten oder geneigten Zustand an der Spannvorrichtung fixierbar ist. Dadurch wird die Einstellbarkeit und Bedienbarkeit der Spannvorrichtung noch weiter verbessert. Alternativ kann anstelle eines ortsveränderlich fixierbaren Griffs im Bereich der Seitenelemente ein, vorzugsweise rastbaren, Aufrollmechanismus für die jeweiligen Zugseile vorgesehen sein.

Seitenelement und/oder Zentralelement weisen Verbindungsmittel auf, um mit der Orthese, gegebenenfalls lösbar, verbunden zu werden. Die Erfindung erlaubt so die Auf- beziehungsweise Abrüstung der Spannvorrichtung auch auf eine bestehende Orthese. Besonders ist die bestehende Orthese eine herkömmliche Gestrickorthese, besonders eine Staborthese, die aufgrund ihrer Eigenelastizität um das Köperteil herumgeführt und in an sich bekannter Weise über Laschen geschlossen wird. Im Falle einer Rückenorthese kann die erfindungsgemäße Spannvorrichtung im Bereich der Rückenseite und der Hüftseite auf die Orthese aufgerüstet werden.

Die erfindungsgemäße Spannvorrichtung erlaubt eine individuelle Anpassung der Spannkraft einer Orthese über einen weiten Bereich. Da die stabilisierende beziehungsweise fixierende Wirkung der Orthese im Wesentlichen vollständig durch die Spannvorrichtung selbst übernommen wird, braucht eine darunterliegende elastische in an sich bekannter Weise ausgebildete Gestrickorthese selbst keine vollständige Spann- oder Stützwirkung mehr ausüben. Es kann daher vorteilhafterweise ein Gestrick verwendet werden, das über einen großen Umfangsbereich, im Falle einer Rückenorthese beispielsweise sowohl bei schmaler Taille als auch bei großem Bauchbeziehungsweise Brustumfang, eingesetzt werden kann, ohne dass jeweils individuell an die Körpergröße angepasste Gestrickorthesen bereitgestellt werden müssen. Die Anpassung an den Körperumfang kann dabei jeweils bevorzugt ausschließlich über die Einstellung der Flaschenzüge, besonders also über die segmentweise Einstellung der "Arbeitspunkte" der losen Enden der Flaschenzüge, vorgenommen werden.

Ein weiterer Gegenstand der Erfindung ist auch eine Zugseilklemme zum individuellen Klemmen zweier Zugseile in einem gemeinsamen Griff, insbesondere zur Verwendung im Zusammenhang mit erfindungsgemäßen Spannvorrichtung in einer Ausgestaltung, worin zumindest zwei Zugseilenden zweier benachbarter Flaschenzüge beabstandet in dem gemeinsamen Griff enden. Die Zugseilklemme ist erfindungsgemäß an oder in dem Griff vorgesehen und gestattet, die wirksame Länge des Zugseils des ihm zugeordneten Spannsegments individuell und unabhängig von der Länge des Zugseils des benachbarten Spannsegments einzustellen, um so eine gezielte Vorspannung oder Entspannung eines bestimmten Spannsegments gegenüber dem benachbarten Spannsegments zu ermöglichen. Auf diese Weise kann die Spannvorrichtung der weiteren Verbesserung der Stützfunktion einer Orthese dienen.

Die erfindungsgemäße Zugseilklemme weist zumindest zwei voneinander beabstandete Zugseilaufnahmen, jeweils zum Aufnehmen eines Zugseilendes, auf. Die Zugseilaufnahmen sind erfindungsgemäß jeweils so ausgestaltet, dass sie ein lösbares Verklemmen des Zugseils in dem Griffstück ermöglichen.

In einer bevorzugten Ausgestaltung weist die Klemmvorrichtung zwei voneinander beabstandete Zugseilaufnahmen auf, um jeweils die Klemmung zweier zueinander beabstandeter Zugseile zu bewirken Dazu ist die Zugseilklemme bevorzugt zweiteilig ausgebildet und setzt sich aus einem Grundelement mit davon ausgebildeter Zugseilaufnahme und einer über das Grundelement steckbaren Kappe zusammen. Kappe und Grundelement kommen im zusammengesteckten Zustand dabei derart in Eingriff, dass das Zugseil in der Zugseilaufnahme des Grundelements durch Verklemmen lösbar fixiert wird. Dazu weist das Grundelement mindestens einen Klemmkonus auf, an dem entlang das Zugseil geführt wird. Dies wird bevorzugt dadurch bewerkstelligt, dass der Klemmkonus eine Durchbrechung oder Bohrung aufweist, wodurch das Seil geführt wird, sodass es beim Austritt aus der Durchbrechung oder Bohrung an der Oberfläche des Klemmkonus entlanggeführt werden kann. Der Klemmkonus des Grundkörpers kommt im zusammengesetzten Funktionszustand der Klemmvorrichtung mit einer in der Kappe vorgesehenen Bohrung, Nut oder Aussparung derart in Kontakt und in Eingriff, dass das an dem Klemmkonus entlanggeführte Zugseil an dessen bevorzugt strukturierter Oberfläche festgeklemmt wird. Bevorzugt weist die Kappe dazu eine konische Bohrung auf, deren Abmessungen mit den äußeren Abmessungen des Klemmkonus des Grundelements korrespondieren.

Um die Kappe an dem Grundkörper lösbar zu fixieren, weist der Grundkörper zumindest ein Rastelement auf, welches mindestens eine oder bevorzugt mehrere Rastungen trägt, die mit entsprechend gestalteten Widerlagern in der Kappe in Eingriff kommen und die Kappe gegen mechanischen Widerstand und äußere Krafteinwirkung an dem Grundelement festhalten. Zum Lösen der Kappe von dem Grundelement zur Verstellung der Zugseillänge wird die Kappe entgegen den mechanischen Widerstand der Rastung in der Kappe abgezogen.

In einer besonderen Ausgestaltung des Griffs zur Aufnahme ist die vorgeschriebene Klemmvorrichtung in einer aus Gewebematerial gefertigten Tasche gelagert. Die Tasche wird zur Benutzung des Griffs geschlossen und die Klemmvorrichtung bleibt während der Benutzung des Griffs verdeckt. Die Fixierung des Griffs an der Orthese oder der Spannvorrichtung findet über die Grifftasche statt.

Die Erfindung wird durch die nachfolgenden Figuren und Figurenbeschreibungen näher illustriert, ohne dass diese beschränkend zu verstehen wären.

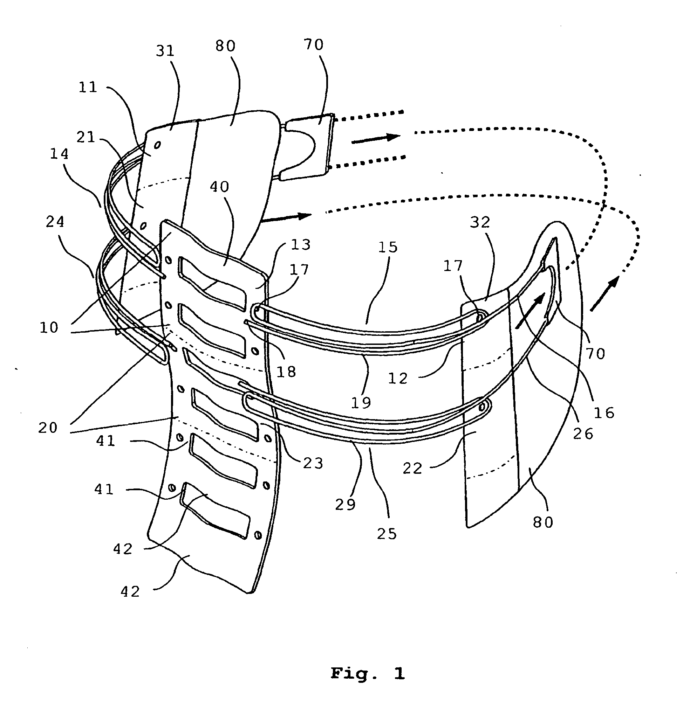

Figur 1 zeigt eine Ausgestaltung der erfindungsgemäßen Spannvorrichtung, angepasst zur Verwendung an einer Rückenorthese zur Stabilisierung der Wirbelsäule. Die Ansicht zeigt aus Gründen der Übersichtlichkeit nicht vollständig alle wiederkehrenden Strukturen. Pro Spannsegment (10) sind, rechts und links des Zentralelements (13), jeweils zwei Zugseilantriebe (14, 15) in Form von Flaschenzügen verbunden, die jeweils mit dem, in der Figur linksseitig und im Hintergrund dargestellten, Seitenelement (11) und dem, rechtsseitig und im Vordergrund dargestellten, zweiten Seitenelement (12) mechanisch verbunden sind. Die Flaschenzüge (14, 15) weisen jeweils Zugseilumlenkungen (17) und Zugseilverankerungen (18) auf, um jeweils einen mehrfachen Flaschenzug zu bilden. Pro Spannsegment sind die Flaschenzüge (14, 15) so in jeweils genau einem Kraftansatzpunkt (17, 18) an Zentralelement (13) und Seitenelement (11, 12) verankert. In der Figur sind die zu Seitenbrücken (31, 32) verbundenen Seitenelemente und zu einer Zentralbrücke (40) verbundenen Zentralelemente über Trennlinien voneinander unterscheidbar gemacht. Die Flaschenzüge (14, 15) des ersten Spannsegments (10) weisen jeweils lose Seilenden (16) auf, über die die Flaschenzüge gespannt werden können, wodurch sich der Abstand zwischen dem Zentralelement (13) und den Seitenelementen (11, 12) jeweils verkürzt, um zu spannen.

Entsprechend weisen die benachbarten Spannsegmente (20) Flaschenzüge (24, 25) auf mit Zugseilen (29) und losen Enden (26). Die Flaschenzüge (24, 25) verbinden jeweils die ersten und zweiten Seitenelemente (21, 22) über das Zentralelement (23) miteinander.

Die untereinander liegenden separaten Spannsegmente (10, 20) sind mechanisch miteinander verkoppelt. Dabei ist jeweils das erste Seitenelement (11) des ersten Spannsegments (10) mit dem ersten Seitenelement (21) des zweiten Spannsegments (20) zu einer gemeinsamen einstückigen Seitenbrücke (31) verkoppelt. Entsprechend ist das zweite Seitenelement (21) des ersten Spannsegments mit dem zweiten Seitenelement (22) des zweiten Spannsegments (20) zu einer einstückigen Seitenbrücke (32) verkoppelt. Ebenso ist das Zentralelement (13) des ersten Spannsegments (10) mit dem Zentralelement (23) des zweiten Spannsegments zu einer einstückigen Zentralbrücke (40) verkoppelt. Trotz der mechanischen Verkopplung mechanischer Spannsegmente (10, 20) miteinander über die Seitenbrücken (31, 32) und die Zentralbrücke (40) ist über die segmentweise getrennt geführten Flaschenzüge eine segmentweise individuelle Anpassung der Spannkraft möglich.

In der Ausgestaltung als Rückenorthese bildet die Zentralbrücke (40) eine anatomisch anformbare Wirbelsäulenstütze, die im angelegten Zustand der Orthese auf oder im Bereich der Wirbelsäule platziert ist. Dazu ist in dieser Ausgestaltung weiter vorgesehen, in der Zentralbrücke (40) Durchbrechungen (41) und, alternativ oder zusätzlich, Ausbuchtungen (42) vorzusehen, um die Druckbeaufschlagung der Wirbelsäule auf Orte, auf Bereiche links und rechts der Wirbelsäule, zu verteilen und eine direkte Druckbeaufschlagung der Wirbelkämme zu verhindern.

Die rechtsseitigen und linksseitigen losen Enden (16, 26) der Flaschenzüge einzelner Spannsegmente (10, 20) münden, zueinander beabstandet, in einen gemeinsamen Griff (70). Der Griff (70) ist im Bereich der Seitenelemente/Seitenbrücken (11, 12, 31, 32) lösbar fixierbar. Durch Verschwenken des Griffs (70) beim Fixieren kann die Spannkraft auf die beiden Spannsegmente individuell verteilt werden. Die gebildeten Seitenbrücken (31, 32) laufen in der dargestellten Ausgestaltung jeweils in Verschlusslaschen (80) aus, die beim Anlegen der Orthese am Körper in Form eines Gürtels oder einer Bandage miteinander kraftschlüssig verbunden werden, wodurch der erfindungsgemäße segmentweise zirkuläre Kraftschluss erreicht wird.

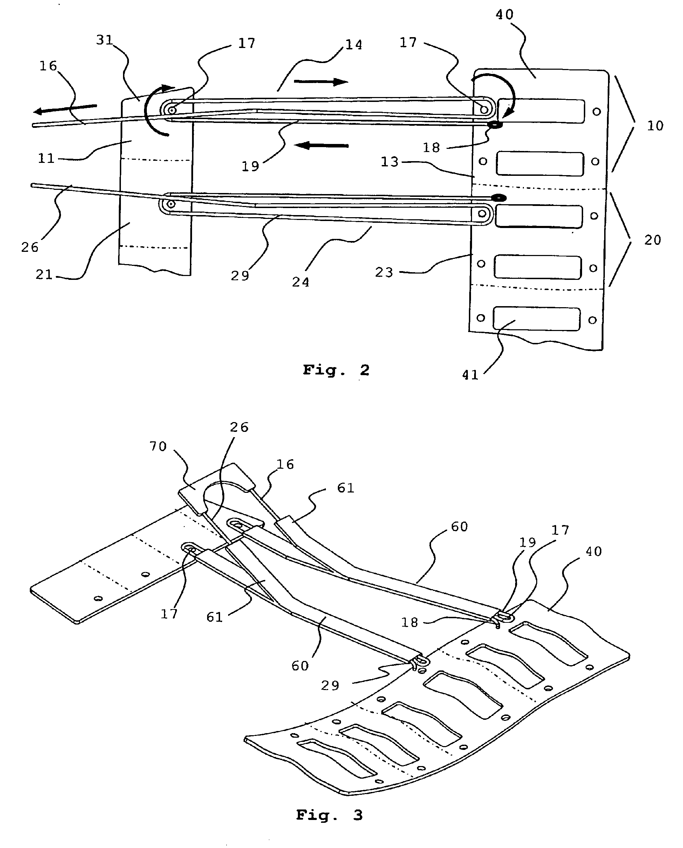

Figur 2 zeigt eine schematische Detaildarstellung einer besonderen Ausgestaltung der erfindungsgemäßen Spannvorrichtung. Es ist nur eine Seite (linke Seite) der Spannvorrichtung dargestellt. Rechtsseitig vom Zentralelement ist die Spannvorrichtung dazu spiegelsymmetrisch ausgebildet. Die Zentralelemente (13, 23) einzelner Spannsegmente (10, 20) sind zu einer integralen Zentralbrücke (40) verkoppelt. Die punktierten Trennlinien zeigen die Koppelstellenspannsegmente an. In der Darstellung ist die Zentralbrücke (40) einstückig ausgebildet. Ebenso sind die ersten Seitenelemente (11, 21) der Spannsegmente (10, 20) zu einer einstückigen Seitenbrücke (31) verkoppelt. Pro Spannsegment ist jeweils genau ein Flaschenzug (14) zwischen Zentralelement (13) und Seitenelement (11) ausgebildet. Ein Zentralelement (13) und/oder ein Seitenelement (11) kann alternative Orte für die Zugseilverankerungspunkte (18) und die Zugseilumlenkelemente (17) vorsehen. Auf diese Weise ist eine individuelle Anpassung der Zugrichtung innerhalb jedes einzelnen Spannsegments möglich. Die Zugseile (19) des Flaschenzugs (14) des Spannsegments (10) verlaufen in unmittelbarer Nachbarschaft oder übereinander liegend aber räumlich getrennt und beabstandet von den Zugseilen (29) des Flaschenzugs (24) eines benachbarten Spannsegments (20). Aus Gründen der Übersichtlichkeit sind auch hier die Flaschenzüge weiterer benachbarter Spannsegmente (30) nicht dargestellt.

Gemäß Figur 3 werden die Zugseile (19, 29) jeweils in Zugseiltunneln (60) geführt. Die Zugseile (19, 29) sind in der dargestellten Ausführung am Ende jeweils aus den Zugseiltunneln (60) herausgeführt, um über die Zugseilumlenkungen (17) geführt beziehungsweise an den Zugseilverankerungen (18) verankert zu werden. Von den jeweiligen Zugseiltunneln (60) zweigt jeweils ein Tunnelzweig (61) ab, worin jeweils die Zugseilenden (16, 26) geführt werden. Diese münden in der dargestellten Form in einem gemeinsamen Griff (70) zum Spannen beider Zugseile.

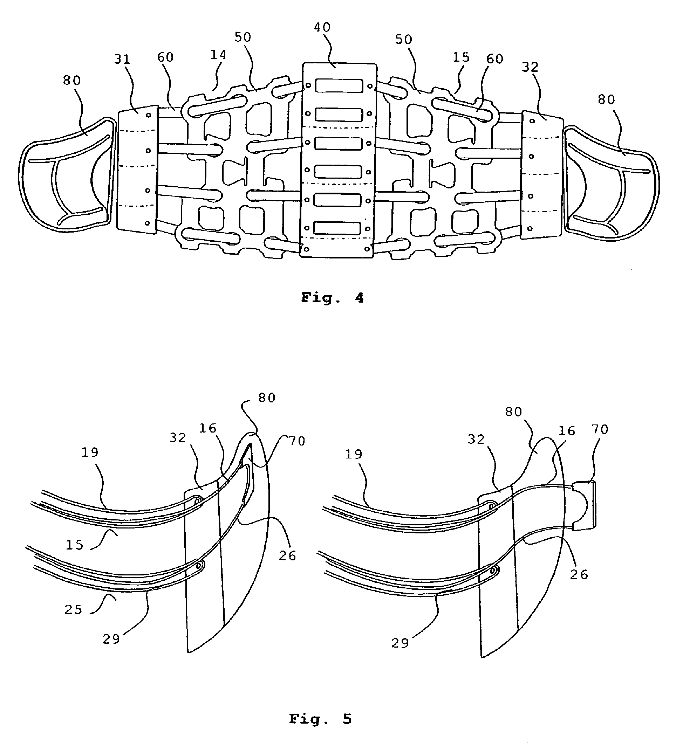

Figur 4 zeigt eine schematische Darstellung einer besonderen Ausführung der erfindungsgemäßen Spannvorrichtung, die auf eine herkömmliche Gestrickorthese aufrüstbar ist. Die zu Seitenbrücken (31, 32) verbundenen Seitenelemente und die zu einer Zentralbrücke (40) verbundenen Zentralelemente bilden im Wesentlichen die mechanisch starren, die Gesamtstruktur der Spannvorrichtung erhaltenden Elemente. Die Zentralbrücke (40) ist gleichzeitig als Stützelement ausgebildet, das im angelegten Zustand der dargestellten Rückenorthese beiderseits der Wirbelkämme positioniert wird; ein unmittelbarer Druck auf die Wirbelkämme wird so vermieden. Die Zugseile der vom Zentralelement zu den Seitenelementen verlaufenden Flaschenzüge (14, 15) laufen in Zugseiltunneln (60) aus elastischem flexiblen Material. Die Zugseiltunnel werden zusätzlich durch die beidseitig zwischen den Zentralelementen und den Seitenelementen angebrachten Gitterrahmen (50) geführt und voneinander beabstandet gehalten. Die Gitterrahmen (50) sind in der dargestellten Ausführung aus steifem Material ausgebildet und dienen gleichzeitig als Beckenstütze. Die Spannvorrichtung nach Figur 4 läuft in beidseitigen Verschlusslaschen (80) aus. Diese können Bestandteil der Spannvorrichtung selbst oder alternativ unmittelbar Bestandteil der aufzurüstenden herkömmlichen Gestrickorthese sein. In einer besonderen Ausgestaltung der Ausführung nach Figur 4 ist die Spannvorrichtung mit den beidseitig an den Seitenbrücken (31, 32) anschließenden Verschlusslaschen (80) selbst unmittelbar als Orthese einsetzbar, ohne dass eine darunterliegende herkömmliche Gestrickorthese verwendet ist. Insofern zeigt Figur 4 bereits eine unmittelbar einsetzbare Orthese, welche die erfindungsgemäße Spannvorrichtung enthält und im Wesentlichen daraus besteht.

Figur 5 zeigt eine Detaildarstellung der Anwendung des Griffs (70) zum gleichzeitigen Spannen der Zugseile (19, 29) der Flaschenzüge (15, 25) benachbarter Spannsegmente über deren in ein gemeinsames Griffstück (70) auslaufenden losen Enden (16, 26). Im gespannten oder gelösten Zustand ist der Griff (70) in der dargestellten Ausführung an der dem Seitenelement (32) zugeordneten und mit diesem mechanisch verbundenen Verschlusslasche (80) lösbar fixiert. Die Fixierung erfolgt beispielsweise über an dem Griff (70) auf der zur Verschlusslasche (80) zugewandten Seite vorhandenen Kletthaken, die in der Gewebeoberfläche der Verschlusslasche (80) eingreifen. Zum Spannen oder Lösen der Spannung wird der Griff (70) von der Verschlusslasche (80) abgehoben. Soll die Spannwirkung eines der beiden dargestellten Spannsegmente gezielt erhöht werden, so wird der Griff (70) derart verschwenkt, dass der Zug an dem jeweiligen losen Ende des Zugseils des entsprechenden Spannsegments im Vergleich zu dem Zug auf das lose Ende des Flaschenzugs des benachbarten Spannsegments verstärkt wird beziehungsweise der Zug auf das andere lose Ende verringert wird.

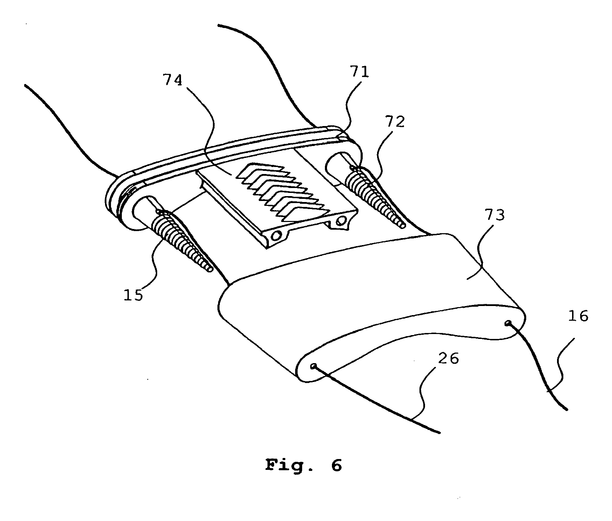

Figur 6 zeigt eine Ausgestaltung des Griffs (70) als Klemmvorrichtung zur lösbaren Fixierung der Zugseilenden (16, 26) zweiter benachbarter Flaschenzüge. Die Klemmvorrichtung besteht aus einem Grundelement (71), woran zwei zueinander beabstandete Klemmkonen (72) ausgebildet sind. Die Klemmkonen (72) weisen Bohrungen zur Aufnahme der Zugseile auf. Weiter ist eine Kappe (73) vorgesehen, die über das Grundelement (71) geschoben wird, wodurch die Zugseile an den Klemmkonen (72) verklemmt werden. Die Kappe (73) ist über ein am Grundkörper gebildetes Rastelement (74) und entsprechend gestalteten Widerlagern in der Kappe (73) (nicht dargestellt) verrastbar.

## Patentansprüche

1. Spannvorrichtung für eine Orthese, enthaltend mehrere separate, unabhängig spannbare Spannsegmente (10), die jeweils ein erstes Seitenelement (11) an einem ersten Ende und ein zweites Seitenelement (12) an einem gegenüberliegenden zweiten Ende und ein dazwischen angeordnetes Zentralelement (13) aufweisen, wobei erstes und zweites Seitenelement (11,12) eines Spannsegments (10) über das Zentralelement (13) dieses Spannsegments durch einen zwischen Zentralelement (13) und erstem Seitenelement (11) verlaufenden ersten Flaschenzug (14) und einen zwischen Zentralelement (13) und zweitem Seitenelement (12) verlaufenden zweiten Flaschenzug (15) miteinander verbunden sind.

2. Spannvorrichtung nach Anspruch 1, wobei die Spannwirkung des Spannsegments (10) durch Verringerung des Abstands der Seitenelemente (11,12) zueinander über die Flaschenzüge (14,15) erzielbar ist.

3. Spannvorrichtung nach Anspruch 2, wobei der Flaschenzug (14,15) durch Ziehen eines losen Endes (16) des Zugseils des Flaschenzugs (14, 15) verkürzbar ist.

4. Spannvorrichtung nach einem der vorstehenden Ansprüche, wobei der Flaschenzug (14,15) als einfacher oder mehrfacher Flaschenzug mit mindestens einem Zugseilumlenkung (17) und einem Zugseilverankerung (18) ausgebildet ist.

5. Spannvorrichtung nach einem der vorstehenden Ansprüche, wobei in einem Spannsegment (10) zwischen erstem Seitenelement (11) und Zentralelement (13) und zwischen zweitem Seitenelement (12) und Zentralelement (13) jeweils genau ein Flaschenzug (14,15) angeordnet ist.

6. Spannvorrichtung nach einem der vorstehenden Ansprüche, wobei das erste Seitenelement (11) eines ersten Spannsegments (10) mit dem ersten Seitenelement (21) eines benachbarten zweiten Spannsegments (20) zu einer ersten integralen Seitenbrücke (31) und das zweite Seitenelement (12) eines ersten Spannsegments (10) mit dem zweiten Seitenelement (22) eines benachbarten zweiten Spannsegments (20) zu einer zweiten integralen Seitenbrücke (32) verbunden sind.

7. Spannvorrichtung nach einem der vorstehenden Ansprüche, wobei das Zentralelement (13) eines ersten Spannsegments (10) mit den dem Zentralelement (23) eines benachbarten zweiten Spannsegments (20) zu einer integralen Zentralbrücke (40) verbunden sind.

8. Spannvorrichtung nach einem der vorstehenden Ansprüche, wobei die Zugseile (19) des Flaschenzugs (14,15) eines ersten Spannsegments (10) beabstandet von den Zugseilen (29) des Flaschenzugs (24,25) eines benachbarten zweiten Spannsegments (20) verlaufen.

9. Spannvorrichtung nach Anspruch 8, wobei die Zugseile (19, 29) jeweils in einem zwischen Zentralelement (13) und Seitenelementen (11,12) angeordneten Gitterrahmen (50) verlaufen.

10. Vorrichtung nach einem der vorstehenden Ansprüche, wobei die Zugseile (19) des Flaschenzugs (14,15) in einem zwischen Seitenelement (11,12) und Zentralelement (13) verlaufenden Zugseiltunnel (60) aus plastischem flexiblem Werkstoff verlaufen.

11. Spannvorrichtung nach Anspruch 10, wobei der Zugseiltunnel (60) in seinem Verlauf zwischen Seitenelement und Zentralelement einen zusätzlichen Tunnelzweig (61) aufweist, worin das lose Ende (16) des Zugseils geführt wird.

12. Spannvorrichtung nach einem der vorstehenden Ansprüche, wobei das lose Ende (16) des Zugseils in einen Griff (70) zum Spannen des Zugseils mündet und darin fixiert sind, wobei der Griff (70) an oder im Bereich der Spannvorrichtung (10) lösbar fixierbar ist.

13. Spannvorrichtung nach Anspruch 12, wobei der Griff (70) eine zum Voreinstellen der Zuglänge des Zugseils (16,19) eingerichtete Klemmvorrichtung (71, 72, 73) aufweist.

14. Spannvorrichtung nach einem der vorstehenden Ansprüche, wobei jeweils die losen Enden (16) der Zugseile der Flaschenzüge (14, 24, 15, 25) benachbarter Spannsegmente (10,20) in einen gemeinsamen Griff (75) münden und darin zueinander beabstandet fixiert sind, um durch Verschwenken oder Neigen des gemeinsamen Griffs (75) den Zug auf den dem ersten Spannsegment (10) zugehörigen Flaschenzug (14, 15) und den dem ersten Spannsegment (20) zugehörigen Flaschenzug (24, 25) individuell verteilbar zu machen.

15. Spannvorrichtung für eine Orthese nach einem der vorstehenden Ansprüche, speziell ausgebildet, um über lösbare mechanische Koppelmittel im Bereich der Seitenelemente (11,12) und/oder des Zentralelements (13) auf eine Gestrickorthese aufrüstbar zu sein.

16. Orthese, enthaltend die Spannvorrichtung nach einem der vorstehenden Ansprüche.

17. Verwendung der Spannvorrichtung nach einem der Ansprüche 1 bis 15 zur segmentweisen Steuerung der Spann- oder Stützfunktion einer Orthese.

## Claims

1. A tightening device for an orthosis, containing a plurality of separate tightening segments (10) that can be independently tightened, each segment comprising a first lateral element (11) at a first end, a second lateral element (12) at an opposing second end, and a central element (13) arranged therebetween, wherein the first and second lateral elements (11, 12) of a tightening segment (10) are connected to each other via the central element (13) of this tightening segment by way of a first tackle assembly (14), which extends between the central element (13) and the first lateral element (11), and a second tackle assembly (15), which extends between the central element (13) and the second lateral element (12).

2. The tightening device according to claim 1, wherein the tightening action of the tightening segment (10) can be achieved by reducing the distance of the lateral elements (11, 12) from each other by way of the tackle assemblies (14, 15).

3. The tightening device according to claim 2, wherein the tackle assembly (14, 15) can be shortened by pulling a loose end (16) of the cable of the tackle assembly (14, 15).

4. The tightening device according to any one of the preceding claims, wherein the tackle assembly (14, 15) is designed as single or multiple tackle assembly having at least one cable return (17) and a cable anchoring point (18).

5. The tightening device according to any one of the preceding claims, wherein exactly one respective tackle assembly (14, 15) is arranged in a tightening segment (10) between the first lateral element (11) and the central element (13), and between the second lateral element (12) and the central element (13).

6. The tightening device according to any one of the preceding claims, wherein the first lateral element (11) of a first tightening segment (10) is connected to the first lateral element (21) of an adjoining second tightening segment (20) to form a first integral lateral bridge (31), and the second lateral element (12) of a first tightening segment (10) is connected to the second lateral element (22) of an adjoining second tightening segment (20) to form a second integral lateral bridge (32).

7. The tightening device according to any one of the preceding claims, wherein the central element (13) of a first tightening segment (10) is connected to the central element (23) of an adjoining second tightening segment (20) to form an integral central bridge (40).

8. The tightening device according to any one of the preceding claims, wherein the cables (19) of the tackle assembly (14, 15) of a first tightening segment (10) run at a distance from the cables (29) of the tackle assembly (24, 25) of an adjoining second tightening segment (20).

9. The tightening device according to claim 8, wherein the cables (19, 29) respectively run in a lattice frame (50) that is arranged between the central element (13) and the lateral elements (11, 12).

10. The device according to any one of the preceding claims, wherein the cables (19) of the tackle assembly (14, 15) run in a cable tunnel (60) that runs between the lateral element (11, 12) and the central element (13) and is made of plastic flexible material.

11. The tightening device according to claim 10, wherein the course of the cable tunnel (60) between the lateral element and the central element has an additional tunnel branch (61), in which the loose end (16) of the cable is guided.

12. The tightening device according to any one of the preceding claims, wherein the loose end (16) of the cable leads into a handle (70) for tightening the cable and is secured therein, wherein the handle (70) is releasably secured on or in the region of the tightening device (10).

13. The tightening device according to claim 12, wherein the handle (70) has a clamping device (71, 72, 73) that is equipped to pre-set the length of the cable (16, 19).

14. The tightening device according to any one of the preceding claims, wherein the respective loose ends (16) of the cables of the tackle assemblies (14, 24, 15, 25) of adjoining tightening segments (10, 20) lead into a common handle (75) and are secured therein at a distance from each other, so as to allow the pull on the tackle assembly (14, 15) associated with the first tightening segment (10) and the tackle assembly (24, 25) associated with the first tightening segment (20) to be individually distributed by pivoting or inclining the common handle (75).

15. The tightening device for an orthosis according to any one of the preceding claims, which is specifically designed to be added to a knitted orthosis by way of releasable mechanical coupling means in the region of the lateral elements (11, 12) and/or of the central element (13).

16. An orthosis, containing the tightening device according to any one of the preceding claims.

17. Use of the tightening device according to any one of claims 1 to 15 for the segmental actuation of the tightening or supporting function of an orthosis.

## Revendications

1. Dispositif de serrage pour une orthèse, contenant plusieurs segments de serrage séparés (10), pouvant être serrés indépendamment qui présentent chacun un premier élément latéral (11) à une première extrémité et un second élément latéral (12) à une seconde extrémité opposée et un élément central (13) disposé entre eux, dans lequel les premier et second éléments latéraux (11, 12) d'un segment de serrage (10) sont reliés ensemble par le biais de l'élément central (13) de ce segment de serrage par une première poulie (14) s'étendant entre l'élément central (13) et le premier élément latéral (11) et une seconde poulie (15) s'étendant entre l'élément central (13) et le second élément latéral (12).

2. Dispositif de serrage selon la revendication 1, dans lequel l'effet de serrage du segment de serrage (10) peut être obtenu par réduction de l'écart des éléments latéraux (11, 12) l'un par rapport à l'autre par le biais des poulies (14, 15).

3. Dispositif de serrage selon la revendication 2, dans lequel la poulie (14, 15) peut être raccourcie par tirage d'une extrémité lâche (16) du câble tracteur de la poulie (14, 15).

4. Dispositif de serrage selon une des revendications précédentes, dans lequel la poulie (14, 15) est réalisée en tant que poulie simple ou multiple avec au moins un renvoi de câble tracteur (17) et un ancrage de câble tracteur (18).

5. Dispositif de serrage selon une des revendications précédentes, dans lequel exactement une poulie (14, 15) est à chaque fois disposée dans un segment de serrage (10) entre le premier élément latéral (11) et l'élément central (13) et entre le second élément latéral (12) et l'élément central (13).

6. Dispositif de serrage selon une des revendications précédentes, dans lequel le premier élément latéral (11) d'un premier segment de serrage (10) est relié au premier élément latéral (21) d'un deuxième segment de serrage voisin (20) en un premier pont latéral intégral (31) et le second élément latéral (12) d'un premier segment de serrage (10) est relié au second élément latéral (22) d'un deuxième segment de serrage voisin (20) en un second pont latéral intégral (32).

7. Dispositif de serrage selon une des revendications précédentes, dans lequel l'élément central (13) d'un premier segment de serrage (10) est relié à l'élément central (23) d'un deuxième segment de serrage voisin (20) en un pont central intégral (40).

8. Dispositif de serrage selon une des revendications précédentes, dans lequel les câbles tracteurs (19) de la poulie (14, 15) d'un premier segment de serrage (10) s'étendent à distance des câbles tracteurs (29) de la poulie (24, 25) d'un deuxième segment de serrage voisin (20).

9. Dispositif de serrage selon la revendication 8, dans lequel les câbles tracteurs (19, 29) s'étendent à chaque fois dans un cadre à mailles (50) disposé entre l'élément central (13) et les éléments latéraux (11, 12).

10. Dispositif selon une des revendications précédentes, dans lequel les câbles tracteurs (19) de la poulie (14, 15) s'étendent dans un tunnel de câble tracteur (60) en matériau flexible plastique s'étendant entre l'élément latéral (11, 12) et l'élément central (13).

11. Dispositif de serrage selon la revendication 10, dans lequel le tunnel de câble tracteur (60) présente une branche de tunnel supplémentaire (61) dans son étendue entre l'élément latéral et l'élément central dans laquelle l'extrémité lâche (16) du câble tracteur est guidée.

12. Dispositif de serrage selon une des revendications précédentes, dans lequel l'extrémité lâche (16) du câble tracteur débouche dans une poignée (70) pour serrer le câble tracteur et y est fixée, dans lequel la poignée (70) peut être fixée de manière amovible à ou dans la région du dispositif de serrage (10).

13. Dispositif de serrage selon la revendication 12, dans lequel la poignée (70) présente un dispositif de fixation (71, 72, 73) installé pour le préréglage de la longueur de traction du câble tracteur (16, 19).

14. Dispositif de serrage selon une des revendications précédentes, dans lequel les extrémités lâches (16) des câbles tracteurs des poulies (14, 24, 15, 25) de segments de serrage voisins (10, 20) débouchent à chaque fois dans une poignée commune (75) et y sont fixées à distance les unes des autres pour rendre la traction sur la poulie (14, 15) appartenant au premier segment de serrage (10) et la poulie (24, 25) appartenant au premier segment de serrage (20) individuellement distribuable par pivotement ou inclinaison de la poignée commune (75).

15. Dispositif de serrage pour une orthèse selon une des revendications précédentes, spécialement réalisé pour pouvoir être monté sur une orthèse tricotée par le biais de moyens de raccordement mécaniques amovibles dans la région des éléments latéraux (11, 12) et/ou de l'élément central (13).

16. Orthèse contenant le dispositif de serrage selon une des revendications précédentes.

17. Utilisation du dispositif de serrage selon une des revendications 1 à 15 pour la commande par segment de la fonction de serrage ou de soutien d'une orthèse.
